# EUROPEAN PATENT APPLICATION

(11) **EP 4 379 050 A1**
(43) Date of publication of application: **05.06.2024**
(21) Application number: 22848384.8
(22) Date of filing: 21.07.2022
(51) Int. Cl.: C12N 9/10, C12N 15/54, C12N 15/63, C12P 19/00

(54) **SUCROSE SYNTHETASE AND USE THEREOF**

(30) Priority: 27.07.2021 CN 202110853028
(71) Applicant: Abiochem Biotechnology (Group) Co., Ltd., Shanghai 200241 (CN)
(72) Inventor: WU, Yan, Shanghai 200241 (CN); TIAN, Zhenhua, Shanghai 200241 (CN); WANG, Shu, Shanghai 200241 (CN); ZHENG, Xiaofu, Shanghai 200241 (CN); XING, Litong, Shanghai 200241 (CN)
(74) Representative: Murgitroyd & Company
(86) International application number: PCT/CN2022/106920
(87) International publication number: WO 2023/005779

(57) **Abstract**

Provided are a sucrose synthetase and the use thereof. Compared with SEQ ID NO: 11, an amino acid sequence of the sucrose synthetase contains differences in amino acid residues selected from one or more of the following residue positions: an amino acid at position 36 being V, an amino acid at position 43 being E, an amino acid at position 179 being A, an amino acid at position 395 being R, an amino acid at position 447 being P, an amino acid at position 455 being L, and an amino acid at position 654 being R; and the sucrose synthetase has an activity not lower than that of a sucrose synthetase having an amino acid sequence as shown in SEQ ID NO: 11. By means of catalyzing the synthesis of rebaudioside A, rebaudioside D or rebaudioside M by using a glucosyltransferase and the sucrose synthetase in a combined manner, a cascade reaction is achieved, thereby providing a variety of possibilities for the selection of raw materials.

## Description

The present application claims the priority of Chinese patent application 202110853028.X filed on July 27, 2021. The contents of the above Chinese patent applications are incorporated herein by reference in its entirety.

### TECHNICAL FIELD

The present disclosure relates to the field of biotechnology, specifically relates to a sucrose synthase and use thereof.

### BACKGROUND

Steviol glycosides are natural sweeteners extracted from the leaves of the Asteraceae herb *stevia rebaudiana.* They are a mixture of various glycosides, and there are significant differences in taste quality among different steviol glycosides. Steviol glycosides possess several advantages: they are pure natural (derived from the pure natural plant *stevia rebaudiana*)*,* highly sweet (250-450 times sweeter than sucrose), low in calories (only 1/300th of white sugar), low cost of use (only one-third of the cost of sucrose), stable (resistant to heat, acids, and alkalis, and less prone to decomposition), and highly safe (non-toxic side effects). Additionally, they have potential therapeutic effects of anti-hyperglycemia, anti-hypertension, anti-inflammation, anti-tumor, and anti-diarrhea.

The structural formula of steviol glycosides (steviol glycoside compounds) is as follows:

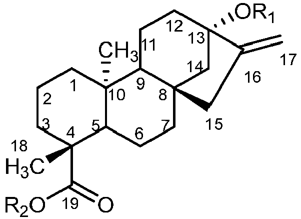

| No. | Compound | R₁ | R₂ |
|---|---|---|---|
| 1 | Steviol | H | H |
| 2 | Steviolmonoside | H | β-Glc |
| 3 | Steviolbioside | H | β-Glc-β-Glc(2-1) |
| 4 | Rubusoside | β-Glc | β-Glc |
| 5 | Stevioside (STV) | β-Glc | β-Glc-β-Glc(2-1) |
| 6 | Rebaudioside A | β-Glc | β-Glc-β-Glc(2-1)-β-Glc(3-1) |
| 7 | Rebaudioside B | H | β-Glc-β-Glc(2-1)-β-Glc(3-1) |
| 8 | Rebaudioside C | β-Glc | β-Glc-α-Rha(2-1)-β-Glc(3-1) |
| 9 | Rebaudioside D | β-Glc-β-Glc(2-1) | β-Glc-β-Glc(2-1)-β-Glc(3-1) |
| 10 | Rebaudioside E | β-Glc-β-Glc(2-1) | β-Glc-β-Glc(2-1) |
| 11 | Rebaudioside F | β-Glc | β-Glc-α-Xly(2-1)-β-Glc(3-1) |
| 12 | Rebaudioside M | β-Glc-β-Glc(2-1)-β-Glc(3-1) | β-Glc-β-Glc(2-1)-β-Glc(3-1) |
| 13 | Dulcoside A | β-Glc | β-Glc-α-Rha(2-1) |

The aforementioned steviol glycosides share a common aglycone: steviol. Their distinction lies in the number and type of glycosyl groups connected at the C-13 and C-19 positions. They primarily include stevioside, rebaudioside A (Reb A, referred to as RA), rebaudioside B, rebaudioside C, rebaudioside D (Reb D, referred to as RD), rebaudioside E, dulcoside, steviolbioside, totaling eight glycosides. The leaves of *stevia rebaudiana* can accumulate up to 10-20% (based on dry weight) of steviol glycosides. The primary glycosides found in the leaves of *stevia rebaudiana* plant are rebaudioside A (2-10%), stevioside (2-10%), and rebaudioside C (1-2%). Other glycosides such as rebaudioside B, D, E, and F, along with steviolbioside and rubusoside, are found in significantly lower levels in the leaves (approximately 0-0.2%).

Although steviol glycosides are high-intensity sweeteners, their application in fields with high taste requirements such as food and beverages, is seriously limited by their lingering bitter aftertaste. The underlying cause of this aftertaste is the intrinsic molecular structure of steviol glycosides. The more glycosyl groups connected to the R₁ and R₂ groups, the better is the taste. Typically, it is found that stevioside is 110-270 times sweeter than sucrose, and rebaudioside A is 150 to 320 times sweeter. However, even in highly purified forms, steviol glycosides still possess undesirable taste attributes, such as bitterness, sweet aftertaste, and licorice flavor, etc.

Rebaudioside D is one of the steviol glycosides with the most potential for application. Compared with other steviol glycosides, its sweetness is high, about 300 to 350 times that of sucrose. It has a pure sweetness and a taste closer to sucrose, without any bitterness or licorice-like off flavor, and good stability, making it an ideal natural high-intensity sweetener. However, the content of rebaudioside D in the leaves of *stevia rebaudiana* is very low (less than 5%), making its production through extraction methods requires a large amount of *stevia rebaudiana* raw material. Additionally, the process of enriching rebaudioside D is complicated, it needs column passes, desalting, decolorization, and recrystallization for several times after extraction. This process generates a considerable amount of wastewater, leading to high production costs, which is not suitable for large-scale industrial production.

Rebaudioside M (Reb M, referred to as RM) has superior taste properties, but its content in the dry weight of leaves is less than 0.1%, leading to high isolation costs and high price. The biocatalytic method to obtain high concentrations of rebaudioside M has attracted the attention of scholars. Currently, it is reported that the recombinant enzymes derived from *stevia rebaudiana* can catalyze rebaudioside D to produce rebaudioside M, but the yield is relatively low. Using rebaudioside D as the substrate, rebaudioside M can be yielded by the microbial enzyme-producing catalysis. Compared with conventional extraction methods, this method not only improves the production process, but also reduces environmental pollution and increases the yield of the desired product, rebaudioside M. The current biological enzyme catalysis method mainly has the following problems: (1) The cost of using biological enzymes to catalyze the production of rebaudioside M from rebaudioside D is relatively high, and the enzyme yield needs to be further optimized; (2) The glycosyltransferase used for catalysis is difficult to be isolated from the product and recycled, and is prone to deactivation; (3) In natural plants, the content of rebaudioside A is quite high, whereas that of rebaudioside D is very low. It is also a difficult problem to be solved urgently to directly convert rebaudioside A into rebaudioside D at low cost.

Glucosyltransferase is an enzyme that only transfers glucosyl groups in enzyme reactions. Its mechanism of action involves catalyzing the transfer of the glucose residues from a glycosyl donor to a glycosyl acceptor molecule, thereby regulating the activity of the acceptor molecule.

"Nucleoside" refers to glycosylamines that contain a nucleobase (i.e., a nitrogenous base) and a 5-carbon sugar (such as ribose or deoxyribose). Non-limiting examples of nucleosides include cytidine, uridine, adenosine, guanosine, thymidine, and inosine.

"Nucleoside diphosphate" refers to a glycosylamine containing a nucleobase (i.e., a nitrogenous base), a 5-carbon sugar (such as ribose or deoxyribose), and a diphosphate (i.e., pyrophosphate) moiety. "Nucleoside diphosphate" can be abbreviated as "NDP". Non-limiting examples of nucleoside diphosphate include cytidine diphosphate (CDP), uridine diphosphate (UDP), adenosine diphosphate (ADP), guanosine diphosphate (GDP), thymidine diphosphate (TDP), and inosine diphosphate (IDP).

UDP-glucose is the abbreviation of uridine diphosphate glucose, also referred to as UDP-Glc or UDPG, it is a vitamin composed of uridine diphosphate and glucose. It can be considered as "active glucose" and is widely distributed in the cells of plants, animals, and microorganisms. It acts as a glucosyl donor in the synthesis of sucrose, starch, glycogen, and other oligosaccharides and polysaccharides, making it the most common type of glycosyl donors. In addition to UDP-glucose, other glycosyl donor compounds such as ADP-glucose, TDP-glucose, dTDP-glucose (deoxythymidine diphosphate glucose), CDP-glucose, IDP-glucose, or GDP-glucose are also commonly used. These glycosyl donors are expensive, which is not conducive to industrial-scale production.

Sucrose synthase (SUS, also referred to as SuSy/SS, EC2.4.1.13) which reversibly catalyzes the chemical reaction of NDP-glucose + D-fructose to NDP and sucrose. It belongs to the glycosyltransferase-4 subfamily and was first identified in wheat (*Triticum aestivum*) germ. Each protein exists in a tetrameric form. The molecular weight of each subunit is around 90 kDa. The size of the gene is typically 5.9 kb, and the size of the cDNA is about 2.7 kb. The encoded amino acid sequence is about 800 residues in full length (Ross and Davies 1992). Sucrose synthase shows varying affinities for different NDPs, with the order of affinity being UDP > ADP > dTDP > CDP > GDP.

The current method for bio-enzymatic synthesis of rebaudioside A, rebaudioside D, or rebaudioside M requires the addition of expensive raw material UDP-glucose or ADP-glucose. This process involves the action of UDP-glucosyltransferase (referred to as UGT), and uses stevioside as the starting material to catalyze the synthesis of rebaudioside A, rebaudioside D, or rebaudioside M. Alternatively, rebaudioside A is used as the starting material to catalyze the synthesis of rebaudioside D or rebaudioside M. However, due to the extremely high price of UDP-glucose, the feasibility of the industrial preparation of rebaudioside D is almost completely limited, resulting in high production costs and lack of market competitiveness.

Nowadays, with the widespread application of the natural sweetener stevioside and the continuous development of biocatalysis technology, glycosyltransferase is increasingly used in the field of biocatalytic preparation of steviol glycosides. There is an urgent need to solve the problem of the high price of the raw material UDPG in order to improve the production efficiency of steviol glycosides. UDPG can be regenerated from sucrose and UDP catalyzed by sucrose synthase. Therefore, it is necessary to modify sucrose synthase to obtain a modified enzyme with higher enzyme activity and better stability, so as to better realize the regeneration of UDPG and realize the industrial-scale production of steviol glycosides.

### CONTENT OF THE PRESENT INVENTION

The technical problem to be solved by the present disclosure is that the existing sucrose synthase has low enzymatic activity and poor stability when applied to the biocatalytic preparation of steviol glycosides, resulting in a low conversion rate when used to catalyze steviol glycosides. Therefore, the present disclosure provides a surcose synthase and use thereof in the preparation of steviol glycosides. The sucrose synthase of the present disclosure exhibits high enzymatic activity and good stability. In the present disclosure, steviosides are used as raw material and jointly used with glucosyltransferase (GT) and sucrose synthase (SUS) to catalyze the synthesis of RD (Rebaudioside D) or RM (Rebaudioside M), realizing a cascade reaction. In the present disclosure, both sucrose and UDP can be used to realize the regeneration of UDPG, as well as sucrose and ADP can be used to realize the regeneration of ADPG, which solves the problem of the high prices of the glycosyl donors UDPG and ADPG. It also provides various possibilities for raw material selection, which provides more options for optimizing process conditions for further realization of large-scale industrial production, and is more conducive to realizing large-scale industrialization.

The first aspect of the present disclosure provides a sucrose synthase, wherein an amino acid sequence of the sucrose synthase comprises differences in amino acid residues selected from one or more of the following residue positions compared to SEQ ID NO: 11:
the amino acid at position 36 is V;
the amino acid at position 43 is E;
the amino acid at position 179 is A;
the amino acid at position 395 is R;
the amino acid at position 447 is P;
the amino acid at position 455 is L;
the amino acid at position 654 is R; and has sucrose synthase activity not lower than that of the amino acid sequence as shown in SEQ ID NO: 11.

The differences in the amino acid sequence of the sucrose synthase can be mutations in SEQ ID NO: 11 or in other sequences, as long as the final result manifests that the amino acid sequence differs from SEQ ID NO: 11 as described above.

Preferably, the amino acid sequence of the sucrose synthase has the following differences in amino acid residues compared to SEQ ID NO: 11:
the amino acid at position 36 is V; or
the amino acid at position 43 is E; or
the amino acid at position 179 is A; or
the amino acid at position 395 is R; or
the amino acid at position 447 is P; or
the amino acid at position 455 is L; or
the amino acid at position 654 is R.

The second aspect of the present disclosure provides an isolated nucleic acid, the nucleic acid encodes the sucrose synthase as described in the first aspect of the present disclosure.

The third aspect of the present disclosure provides a recombinant expression vector comprising the nucleic acid as described in the second aspect of the present disclosure.

The fourth aspect of the present disclosure provides a transformant, the transformant is a host cell comprising the nucleic acid as described in the second aspect of the present disclosure or the recombinant expression vector as described in the third aspect of the present disclosure.

Preferably, the host cell is *Escherichia coli,* for example, *E. coli* BL21 (DE3).

The fifth aspect of the present disclosure provides a method for preparing the sucrose synthase as described in the first aspect of the present disclosure, and the method comprises culturing the transformant as described in the fourth aspect of the present disclosure under conditions suitable for expressing the sucrose synthase.

The sixth aspect of the present disclosure provides a composition comprising one or more of the sucrose synthases as described in the first aspect of the present disclosure.

Preferably, the composition comprises enzymes as shown in SEQ ID NO: 9, 10, and/or SEQ ID NO: 11.

The seventh aspect of the present disclosure provides a method for preparing a glycosyl donor, and the method comprises the following steps:
reacting sucrose with nucleoside diphosphate in the presence of the sucrose synthase as described in the first aspect of the present disclosure to obtain the glycosyl donor; the nucleoside diphosphate is UDP, dTDP, TDP, CDP, IDP, GDP, or ADP;
preferably, the method for preparing the glycosyl donor satisfies one or more of the following conditions:
the sucrose synthase is present in the form of sucrose synthase bacterial cells, crude enzyme solution, purified enzyme, purified enzyme solution, or immobilized enzyme;
a mass ratio of the sucrose synthase bacterial cells to sucrose is 1 :(20-200), preferably 3:200;
a mass ratio of the sucrose to the nucleoside diphosphate is 100:0.5 to 300:0.1; preferably 200:0.1;
a concentration of the sucrose is preferably 50-300 g/L, for example, 200 g/L.

In one preferred embodiment of the present disclosure, the method for preparing the sucrose synthase bacterial cells is as follows: (1) genetically engineered bacteria containing the point-mutated sucrose synthase are cultivated until OD₆₀₀ reaches 0.5 to 1.0, for example, approximately 0.8. IPTG is then added to a final concentration of 0.1 to 0.3 mM, for example, 0.1 mM, and induce the culture at 20°C to 28°C, for example, 25°C, for 16 to 26 h, for example, 20 h. (2) after cultivation, the culture solution is centrifuged at 3000 to 5000 rpm, for example, 4000 rpm, for 10 to 30 min, for example, 20 min. The supernatant is discarded, and the bacterial cells are collected. The collected bacterial cells are then resuspended in PBS (20 to 100 mM, for example, 50 mM, pH 5 to 7, for example, pH 6.0) at a ratio of 1:5 to 1:20, for example, 1:10 (M/V, g/mL). The mixture is homogenized using a high-pressure homogenizer (for example, homogenization at 550 Mbar for 1.5 min). The homogenized enzyme solution is centrifuged, for example, at 12000 rpm for 2 min, and the supernatant is collect to obtain the sucrose synthase bacterial cells.

The eighth aspect of the present disclosure provides a method for preparing rebaudioside D, and the method comprises the following steps: in the presence of one or more of the sucrose synthase as described in the first aspect of the present disclosure, SEQ ID NO: 9, 10, and SEQ ID NO: 11, reacting rebaudioside A, glycosyltransferase, sucrose, and nucleoside diphosphate to obtain rebaudioside D;
preferably, the method for preparing rebaudioside D satisfies one or more of the following conditions:
the sucrose synthase is present in the form of sucrose synthase bacterial cells, crude enzyme solution, purified enzyme, purified enzyme solution, or immobilized enzyme;
the glycosyltransferase is present in the form of glycosyltransferase bacterial cells, crude enzyme solution, purified enzyme, purified enzyme solution, or immobilized enzyme;
a concentration of the rebaudioside A is 1-150 g/L, preferably 50 g/L or 60 g/L;
a concentration of the sucrose is preferably 50-300 g/L, for example, 200 g/L;
a mass ratio of the sucrose synthase bacterial cells to sucrose is 1 :(20-200), preferably 3:200;
more preferbaly, a mass ratio of the glycosyltransferase bacterial cells to the rebaudioside Ais (0.1-2): 1, for example, 0.24: 1 or 0.3:1;
a reaction solvent for the reaction is water, with a pH of 5 to 8, preferably at pH 6; the pH is controlled by a buffer solution, preferably a phosphate buffer solution;
a rotation speed during the reaction is 500-1000 rpm, preferably 600 rpm;
a temperature of the reaction system during the reaction is 20-90°C, preferably 60°C.
Preferably, a molar ratio of the glycosyl donor to rebaudioside A is 1: 1 to 5:1;
the glycosyl donor is obtained by the method described in the seventh aspect of the present disclosure.

The ninth aspect of the present disclosure provides a method for preparing rebaudioside M, which comprises the steps of preparing rebaudioside D as described in the eighth aspect of the present disclosure.

The tenth aspect of the present disclosure provides a use of one or more of the sucrose synthases as described in the first aspect of the present disclosure, SEQ ID NO: 9, 10, and/or SEQ ID NO: 11, in the preparation of steviol glycosides.

Preferably, the steviol glycoside is rebaudioside A, D, or M.

The positive progressive effects of the present disclosure are as follows:

The sucrose synthase of the present disclosure exhibits high enzymatic activity and good stability. When it is used to prepare steviol glycoside (for example, rebaudioside D or M), the catalytic activity and conversion rate are significantly improved compared to those of the sucrose synthase parent; in the present disclosure, stevioside is used as raw material, and jointly used with glucosyltransferase (GT) and sucrose synthase (SUS) to catalyze the synthesis of synthesize RD (rebaudioside D) or RM (rebaudioside M), realizing a cascade reaction. The sucrose synthase of the present disclosure can use ADP and UDP as substrates, among which ADP has the highest activity, that is, in the present disclosure, both sucrose and UDP can be used to realize the regeneration of UDPG (UDP-glucose), as well as sucrose and ADP can be used to realize the regeneration of ADPG (ADP-glucose), which solves the problem of the high prices of the glycosyl donors UDPG and ADPG. It also provides various possibilities for raw material selection, which provides more options for optimizing process conditions for further realization of large-scale industrial production, and is more conducive to realizing large-scale industrialization.

### BRIEF DESCRIPTION OF THE DRAWINGS

Figure 1 presents a schematic diagram of the route for preparing rebaudioside A, D, and M from stevioside, according to one embodiment of the present disclosure
Figure 2 presents a chromatogram of the reference substance of the substrate rebaudioside A (with a retention time of 14.186 min).
Figure 3 presents a chromatogram of the reference substance of the product rebaudioside D (with a retention time of 11.821 min).
Figure 4 presents a chromatogram of the reference substance of the product rebaudioside M (with a retention time of 12.316 min).
Figure 5A and 5B present the HPLC chromatogram of the activity of the p arent enzyme Enz.8 in catalyzing the synthesis of RD (with ADP as the substrate, reaction time 30 min) in the second round of screening as listed in Table 7.
Figure 6A and 6B present the HPLC chromatogram of the activity of the mutant Enz.11 in catalyzing the synthesis of RD (with ADP as the substrate, reaction time 30 min) in the second round of screening as listed in Table 7.
Figure 7A and 7B present the HPLC chromatogram of the activity of th e p arent enzyme Enz.8 in catalyzing the synthesis of RM (reaction time 7 h) as listed in Table 8.
Figure 8A and 8B present the HPLC chromatogram of the activity of Enz. 1 1 in catalyzing the synthesis of RM (reaction time 7 h) as listed in Table 8.

### DETAILED DESCRIPTION OF THE PREFERRED EMBODIMENT

The present disclosure is further illustrated by way of examples, but it is not limited to the scope of these examples. Experimental methods that do not indicate specific conditions in the following examples were selected according to conventional methods and conditions, or according to the product's instruction manual.

Unless otherwise specified, the experimental methods used in the present disclosure are conventional methods. For specific gene cloning operations, please refer to the "Molecular Cloning: A Laboratory Manual" by J. Sambrook *et al.*

Unless otherwise specified, the abbreviations for amino acids used in the present disclosure are conventional in the field. The specific abbreviated symbols corresponding to the amino acids are shown in Table 1.

**Table 1**

| Names of amino acids | Three-letter symbol s | One-letter symbols | Names of amino acids | Three-letter symbols | One-letter symbols |
|---|---|---|---|---|---|
| Alanine | Ala | A | Leucine | Leu | L |
| Arginine | Arg | R | Lysine | Lys | K |
| Asparagine | Asn | N | Methionine | Met | M |
| Aspartic acid | Asp | D | Phenylalanine | Phe | F |
| Cysteine | Cys | C | Proline | Pro | P |
| Glutanine | Gln | Q | Serine | Ser | S |
| Glutamic acid | Glu | E | Threonine | Thr | T |
| Glycine | Gly | G | Tryptophan | Trp | W |
| Histidine | His | H | Tyrosine | Tyr | Y |
| Isoleucine | Ile | I | Valine | Val | V |

The codons corresponding to the amino acids are also conventional in the field. The specific correspondence between amino acids and codons is shown in Table 2.

**Table 2**

| First nucleotide | Second nucleotide | | | | Third nucleotide |
|---|---|---|---|---|---|
| | T | C | A | G | |
| T | Phenylalanine (F) | Serine (S) | Tyrosine (Y) | Cysteine (C) | T |
| | Phenylalanine (F) | Serine (S) | Tyrosine (Y) | Cysteine (C) | C |
| | Leucine (L) | Serine (S) | Stop codon | Stop codon | A |
| | Leucine (L) | Serine (S) | Stop codon | Tryptophan (W) | G |
| C | Leucine (L) | Proline (P) | Histidine (H) | Arginine (R) | T |
| | Leucine (L) | Proline (P) | Histidine (H) | Arginine (R) | C |
| | Leucine (L) | Proline (P) | Glutamine (Q) | Arginine (R) | A |
| | Leucine (L) | Proline (P) | Glutamine (Q) | Arginine (R) | G |
| A | Isoleucine (I) | Threonine | Asparagine | Serine (S) | T |
| | | (T) | (N) | | |
| | Isoleucine (I) | Threonine (T) | Asparagine (N) | Serine (S) | C |
| | Isoleucine (I) | Threonine (T) | Lysine (K) | Arginine (R) | A |
| | Methionine (M) | Threonine (T) | Lysine (K) | Arginine (R) | G |
| G | Valine (V) | Alanine (A) | Aspartic acid (D) | Glycine (G) | T |
| | Valine (V) | Alanine (A) | Aspartic acid (D) | Glycine (G) | C |
| | Valine (V) | Alanine (A) | Glutamic acid (E) | Glycine (G) | A |
| | Valine (V) | Alanine (A) | Glutamic acid (E) | Glycine (G) | G |

KOD Mix enzyme was purchased from TOYOBO CO., LTD. *Dpnl* enzyme was purchased from Invitrogen (Shanghai) Trading Co., Ltd. *E.coli* Trans10 competent cells and *E.coli* BL21 (DE3) competent cells were both purchased from Beijing Dingguo Changsheng Biotechnology Co., Ltd. pGro7 plasmid was purchased from Shanghai Beinuo Biotech Co., Ltd. Sucrose was purchased from Sangon Biotech (Shanghai) Co., Ltd. Reference substance of Reb A was purchased from Macklin. Reference substances of RebD and RebM were purchased from Qingdao Siyuan Stevia International Trade Co., Ltd.

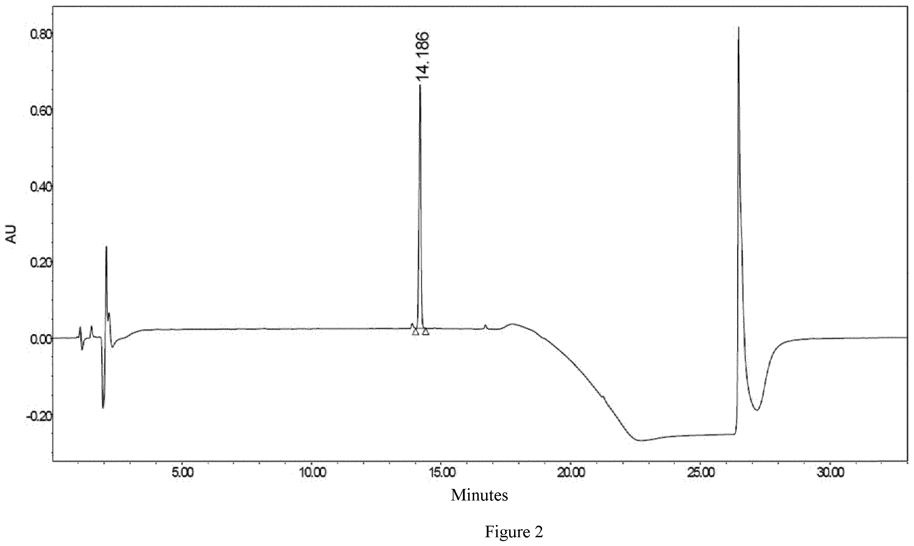

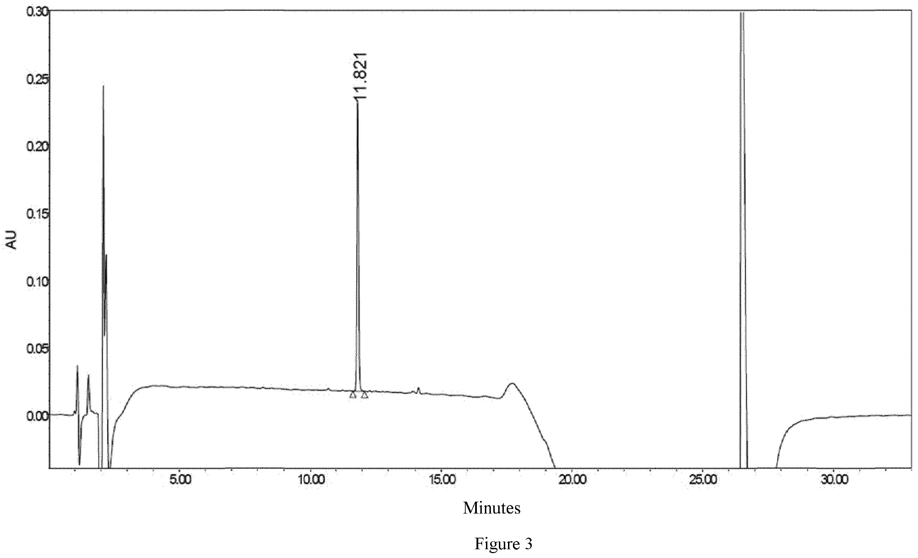

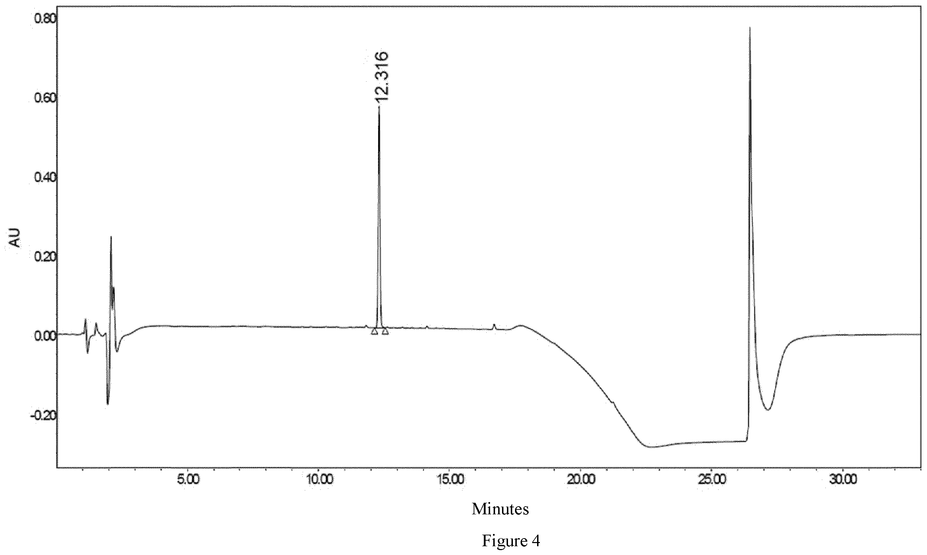

HPLC detection method for conversion rate: chromatographic column: ZORBAX Eclipse Plus C18 (4.6 mm×150 mm, 3.5 µm). Mobile phase: 0.1% TFA aqueous solution as mobile phase A, and 0.1% TFA acetonitrile solution as mobile phase B. Gradient elution is carried out according to Table 3 below. Detection wavelength: 210 nm; flow rate: 1 mL/min; injection volume: 20 µL; column temperature: 35°C. As shown in Figure 2, the peak time of Reb A is 14.186 min; as shown in Figure 3, the peak time of Reb D is 11.821 min. As shown in Figure 4, the peak time of Reb M is 12.316 min.

**Table 3**

| Time (min) | A% | B% |
|---|---|---|
| 0.00 | 90 | 10 |
| 15.00 | 60 | 40 |
| 20.00 | 0 | 100 |
| 24.00 | 0 | 100 |
| 24.10 | 90 | 10 |
| 32.00 | 90 | 10 |

### Example 1: Preparation of sucrose synthase enzymes in the first round

The whole gene synthesis of the sucrose synthase genes numbered Enz.1, Enz.2, Enz.3, Enz.4, Enz.5, Enz.6, Enz.7, and Enz.8, as shown in SEQ ID NO: 1, SEQ ID NO: 2, SEQ ID NO: 3, SEQ ID NO: 4, SEQ ID NO: 5, SEQ ID NO: 6, SEQ ID NO: 7, and SEQ ID NO: 8 listed in Table 4, was carried out. These genes were respectively inserted into the pET28a plasmid vector (restriction sites *Nde*I and *Hind*III) to obtain recombinant plasmids pET28a-Enz.1, pET28a-Enz.2, pET28a-Enz.3, pET28a-Enz.4, pET28a-Enz.5, pET28a-Enz.6, pET28a-Enz.7, and pET28a-Enz.8. And they were sequenced to verify that the genes were successfully ligated into the corresponding vectors. The gene synthesis was carried out by Sangon Biotech Co., Ltd. (Located at No. 698 Xiangmin Road, Songjiang District, Shanghai).

The recombinant plasmids pET28a-Enz.1, pET28a-Enz.2, pET28a-Enz.3, pET28a-Enz.4, pET28a-Enz.5, pET28a-Enz.6, pET28a-Enz.7, pET28a-Enz.8 along with the pGro7 plasmid (which expresses chaperone proteins to increase the solubility of the target protein) were separately co-transformed into host *E. coli* BL21 (DE3) competent cells to obtain engineered strains containing each sucrose synthase gene, respectively. These engineered strains containing each sucrose synthase gene, were activated by plate streaking, respectively. A single colony was selected and inoculated into 5 mL of LB liquid medium containing 50 µg/mL kanamycin and 25 µg/mL chloramphenicol. The culture was then incubated at 37°C for 4 h with shaking. The culture was transferred to 50 mL of fresh TB liquid medium containing also 50 µg/mL kanamycin and 25 µg/mL chloramphenicol with an inoculum volume of 2% (v/v), and then incubated at 37°C with shaking until the OD₆₀₀ reached approximately 0.8. IPTG was then added to a final concentration of 0.1 mM, and the culture was induced at 25°C for 20 h. After cultivation, the culture solution was centrifuged at 4000 rpm for 20 min. The supernatant was discarded, and the bacterial cells were collected and stored at -20°C for later use.

The collected bacterial cells were resuspended in PBS (50 mM, pH 6.0) at a ratio of 1:10 (M/V, g/mL), and then homogenized using a high-pressure homogenizer (homogenization at 550 Mbar for 1.5 min). The homogenized enzyme solution was centrifuged at 12000 rpm for 2 min. The supernatant was collected, and the sucrose synthase reaction enzyme solution was obtained.

**Table 4**

| Enzyme number | Source | NCBI Registration Number | DNA Sequence | Amino Acid Sequence |
|---|---|---|---|---|
| Enz.1 | *Arabidopsis thaliana* | NP_001031915.1 | SEQ ID | / |
| | | | NO: 1 | |
| Enz.2 | *Solanum tuberosum* | P10691.1 | SEQ ID NO: 2 | / |
| Enz.3 | *Thermosynechococcus elongatus* | WP_011056890.1 | SEQ ID NO: 3 | SEQ ID NO: 9 |
| Enz.4 | *Acidithiobacillus ferrianus* | WP_163099567.1 | SEQ ID NO: 4 | / |
| Enz.5 | *Nitrosomonas europaea* | Q820M5.1 | SEQ ID NO: 5 | SEQ ID NO: 10 |
| Enz.6 | *Melioribacter roseus P3M-2* | I7A3T6.1 | SEQ ID NO: 6 | / |
| Enz.7 | *Denitrovibrio acetiphilus DSM 12809* | D4H6M0.1 | SEQ ID NO: 7 | / |
| Enz.8 | *Methylocaldum szegediense* | WP_156912771.1 | SEQ ID NO: 8 | SEQ ID NO: 11 |

### Example 2: Preparation of β-1,2-glycosyltransferase

According to the gene of β-1,2-glycosyltransferase (enzyme number: Enz.9, those skilled in the field can obtain the amino acid sequence of this enzyme based on the nucleotide sequence as shown in SEQ ID NO: 12) as the nucleotide sequence of SEQ ID NO: 12 shown, a set of the genes of β-1,2-glycosyltransferase was synthesized and then ligated into the pET28a plasmid vector to obtain the recombinant plasmid pET28a-1,2GT. Gene synthesis was conducted by Sangon Biotech (Shanghai) Co., Ltd.

The plasmid pET28a-1,2GT was transformed into host *E*. *coli* BL21 (DE3) competent cells to obtain an engineered bacterial strain containing the β-1,2-glycosyltransferase gene.

After activating the engineered strain containing the β-1,2-glycosyltransferase gene by plate streaking, A single colony was selected and inoculated into 5 mL of LB liquid medium containing 50 µg/mL kanamycin, and then incubated at 37°C for 12 h with shaking. The culture was transferred to 50 mL of fresh LB liquid medium containing 50 µg/mL kanamycin with an inoculum volume of 2% (v/v), and then incubated at 37°C with shaking until the OD₆₀₀ reached 0.6-0.8. Isopropyl-β-D-thiogalactopyranoside (IPTG) was added to a final concentration of 0.1 mM, and the culture was induced at 24°C for 22 h. After cultivation, the culture solution was centrifuged at 10,000 rpm for 10 min. The supernatant was discarded, and the bacterial cells were collected and stored in an ultra-low temperature refrigerator at - 20°C for later use.

A 50 mM, pH 6.0 phosphate buffer solution (PBS) was prepared. The collected bacterial cells were suspended at a ratio of 1:10 (M/V, g/mL), then high-pressure homogenization (550-600 Mbar for 1 minute) was carried out. After homogenization, the suspension was centrifuged at 12,000 rpm for 2 min. The supernatant was collected, and the crude enzyme solution of 0-1,2-glycosyltransferase was obtained.

### Example 3: Screening of sucrose synthase in the first round

Using Reb A (content of 96%, purchased from Bidepharm) as the substrate, 150 µL of reaction enzyme solution of β-1,2-glycosyltransferase was added into 1 mL reaction system, and the final concentration of Reb A is 50 g/L, the final concentration of ADP or UDP is 0.1 g/L, and the final concentration of sucrose is 200 g/L. Then, 30 µL of the sucrose synthase reaction enzyme solution was added. Finally, 50 mM (pH 6.0) phosphate buffer solution was added to a final volume of 1 mL. The prepared reaction system was placed in a metal bath and reacted at 60°C and 600 rpm for 1 h. Afterwards, 10 µL of the reaction solution was added into 190 µL of hydrochloric acid (pH 2.0), the mixture was then vortexed, followed by 800 µL of deionized water was added and mixed well. After the mixture was centrifuged at 13,000 rpm for 10 min, the supernatant was collected for HPLC to analyze the concentration of Reb D (see Table 5 for RD%).

**Table 5**

| Sucrose synthase | RD% (ADP) | RD% (UDP) |
|---|---|---|
| Enz.1 | 0.82 | 2.50 |
| Enz.2 | 0.53 | 0.34 |
| Enz.3 | 15.96 | 13.98 |
| Enz.4 | 1.11 | 0.59 |
| Enz.5 | 18.97 | 8.71 |
| Enz.6 | 10.99 | 1.66 |
| Enz.7 | 4.66 | 0.09 |
| Enz.8 | 38.70 | 7.03 |

The results in Table 5 indicated that when ADP was used as the substrate for sucrose synthase catalysis, Enz.8 had the highest enzyme activity, followed by Enz.5 and Enz.3. When UDP was used as the substrate for sucrose synthase catalysis, Enz.3 had the highest enzyme activity, followed by Enz.5 and Enz.8. Additionally, the activities of Enz.3, Enz.5, and Enz.8 with ADP as the substrate were higher than those with UDP as the substrate, and Enz.8 had the best effect with ADP as the substrate. Therefore, in the next round of experiments, ADP will be used as the substrate for sucrose synthase, and mutations and screenings will be conducted based on Enz.8.

### Example 4: Preparation of sucrose synthase mutants in the second round

Using the recombinant plasmid pET28a-Enz.8 as a template, and the primer sequences shown in Table 6 were used, with Enz.X-F/Km-R and Km-F/Enz.X-R as primers, respectively (wherein Enz.X refers to: Enz.10, Enz.11, Enz.12, Enz.13, Enz.14, Enz.15, and Enz.16). PCR amplification of the target DNA fragments and vector fragments was performed using KOD enzyme.

**Table 6**

| Mutatio n site | Enzym e ID | Primer name | Primer sequence | SEQ ID NO: |
|---|---|---|---|---|
| E36V | Enz.10 | Enz.10-F | AAAAAACTGGTACGCTCCTTCCTTCA GCGT | 13 |
| | | Enz.10-R | AAGGAGCGTACCAGTTTTTTAATCTG GTGC | 14 |
| S43E | Enz. 11 | Enz.11-F | TTCAGCGTGAAGAACTGTGCGACGC GTTTG | 15 |
| | | Enz.11-R | GCACAGTTCTTCACGCTGAAGGAAG GAGCGT | 16 |
| R179A | Enz.12 | Enz.12-F | GGTGGTGAAGCCATCCTGAACTTCCT GCGC | 17 |
| | | Enz.12-R | GTTCAGGATGGCTTCACCACCTTCAC CCAGT | 18 |
| L395R | Enz.13 | Enz.13-F | GTTACACTCGGGATAGCGAACGTGAA CTG | 19 |
| | | Enz.13-R | GTTCGCTATCCCGAGTGTAACGTTCC AGGTA | 20 |
| R447P | Enz.14 | Enz.14-F | ACCAAATACCCTCACGCCGACCTGTT CTGG | 21 |
| | | Enz.14-R | GTCGGCGTGAGGGTATTTGGTTTTTTC CAG | 22 |
| E455L | Enz.15 | Enz.15-F | GTTCTGGCAGCTAAACGAAGCGCAGT ATCAT | 23 |
| | | Enz.15-R | GCTTCGTTTAGCTGCCAGAACAGGTC GGCG | 24 |
| D654R | Enz.16 | Enz.16-F | CTGCCGAAACGTCTGGCGGGTGAATT TTAC | 25 |
| | | Enz.16-R | CCGCCAGACGTTTCGGCAGACGCAG ACCC | 26 |
| / | Univers al primer | Km-F | GCCCGACATTATCGCGAGC | 27 |
| | | Km-R | GGGTATAAATGGGCTCGCG | 28 |

The PCR amplification reaction system is:
KOD Mix: 25 µL
ddHzO: 20 µL
Primer: 2 µL×2
Template: 1 µL.

### PCR procedure:

(1) 98°C 3 min
(2) 98°C 10 s
(3) 55°C 5 s
(4) 68°C 5 s/kbp
(5) 68°C 5 min
(6) hold at 12°C

Steps (2) to (4) are repeated for 34 cycles.

The PCR products were digested using *Dpn*I , and the target DNA fragments were obtained by gel electrophoresis and gel recovery. Two-fragment homologous recombinase (Exnase CE II) purchased from Vazyme Biotech, was used for recombination and ligation. After ligation, the mixture was transformed into *E. coli* Trans 10 competent cells. The cells were then spread onto LB medium containing 50 µg/mL kanamycin, and the plate was incubated upside down at 37°C overnight. A single colony was then picked and inoculated into LB tubes (Km resistance) and incubated for 8-10 h. Plasmids were extracted for sequencing and identification, the recombinant plasmid containing the target mutant gene was obtained.

The recombinant plasmid sequenced correctly was then transformed into the host *E*. *coli* BL21 (DE3) competent cells, an engineered strain containing sucrose synthase gene with point mutations was obtained. The engineered strain containing sucrose synthase gene was activated by plate streaking, and a single colony was selected and inoculated into 5 mL of LB liquid medium containing 50 µg/mL kanamycin. The cultures were incubated at 37°C for 4 h with shaking. The culture was then transferred to 50 mL of fresh TB liquid medium containing 50 µg/mL kanamycin with an inoculum volume of 2% (v/v), and then incubated at 37°C with shaking until the OD₆₀₀ reached approximately 0.8. IPTG was added to a final concentration of 0.1 mM, and the culture was induced at 25°C for 20 h. After cultivation, the culture solution was centrifuged at 4000 rpm for 20 min. The supernatant was discarded, and the bacterial cells were collected and stored at -20°C for later use.

The collected bacterial cells were resuspended in PBS (50 mM, pH 6.0) at a ratio of 1:10 (M/V, g/mL), and then homogenized using a high-pressure homogenizer (homogenization at 550 Mbar for 1.5 min). The homogenized enzyme solution was centrifuged at 12000 rpm for 2 min. The supernatant was collected, and the reaction enzyme solution was obtained.

### Example 5: Screening of sucrose synthase mutant in the second round

Using Reb A (content of 96%) as the substrate, 150 µL of reaction enzyme solution of β-1,2-glycosyltransferase was added into 1 mL reaction system, the final concentration of Reb A is 50 g/L, the final concentration of ADP is 0.1 g/L, and the final concentration of sucrose is 200 g/L. Then, 30 µL of the sucrose synthase reaction enzyme solution was added. Finally, 50 mM (pH 6.0) phosphate buffer solution was added to a final volume of 1 mL. The prepared reaction system was placed in a metal bath and reacted at 60°C and 600 rpm for 30 min. Afterwards, 10 µL of the reaction solution was added into 190 µL of hydrochloric acid (pH 2.0), the mixture was then vortexed, followed by 800 µL of deionized water was added and mixed well. After the mixture was centrifuged at 13,000 rpm for 10 min, the supernatant was collected for HPLC to analyze the concentration of Reb D (RD%); the results are as shown in Table 7.

**Table 7**

| Enzyme | Mutation site | Codon | RD% (30 min) |
|---|---|---|---|
| Enz.10 | E36V | CTA | 25.049 |
| Enz.11 | S43E | GAA | 35.652 |
| Enz.12 | R179A | GCC | 23.447 |
| Enz.13 | L395R | CGG | 33.283 |
| Enz.14 | R447P | CCT | 34.315 |
| Enz.15 | E455L | CTA | 22.093 |
| Enz.16 | D654R | CGT | 35.113 |
| Enz.8 | / | / | 31.212 |

The results showed that within the first 30 min of the reaction, Enz. 11 had the highest enzyme activity, which was increased by 10% compared to the parent enzyme, Enz.8, followed by Enz. 16, Enz. 14, and Enz.13, whose enzyme activities were increased by 5% to 10% compared to parent enzyme Enz.8.

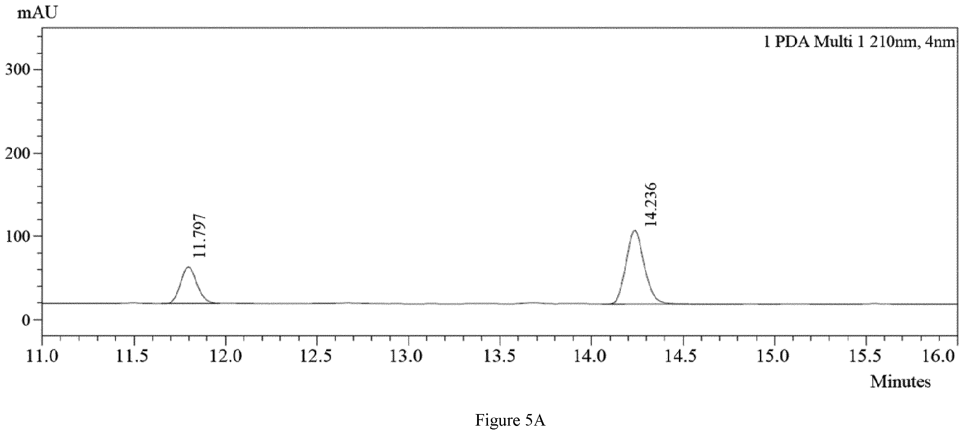

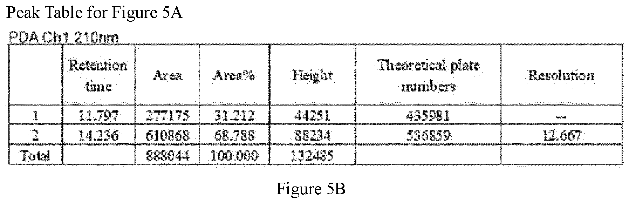

Figure 5A presents the HPLC chromatogram of the parent enzyme Enz.8 catalyzing the synthesis of RD (with ADP as the substrate, 30 min) in the second round of screening as shown in Table 7, while Figure 5B presents the peak information of Figure 5A.

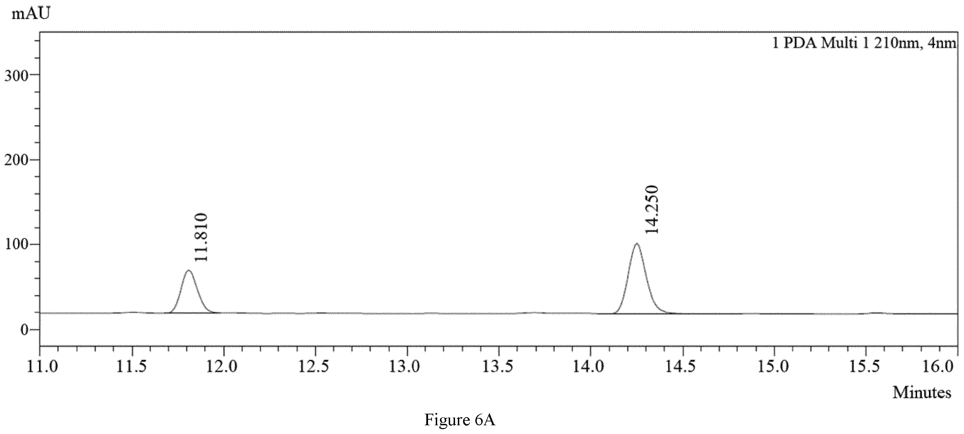

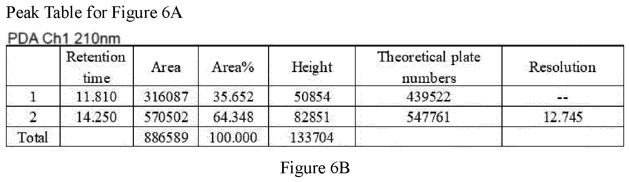

Figure 6A presents the HPLC chromatogram of the mutant Enz.11 catalyzing the synthesis of RD (with ADP as the substrate, 30 min) in the second round of screening as shown in Table 7, while Figure 6B presents the peak information of Figure 6A.

### Example 6: Preparation of β-1,3-glycosyltransferase Enz.17

According to the gene of β-1,3-glycosyltransferase (enzyme number Enz.17, those skilled in the field can obtain the amino acid sequence of this enzyme based on the nucleotide sequence as shown in SEQ ID NO: 29) as the nucleotide sequence of SEQ ID NO: 29 shown, a set of genes of β-1,3-glycosyltransferase was synthesized and then ligated to the pET28a plasmid vector to obtain the recombinant plasmid pET28a-Enz.17. Gene synthesis was conducted by Sangon Biotech (Shanghai) Co., Ltd.

The plasmid pET28a-Enz.17 was transformed into host *E*. *coli* BL21 (DE3) competent cells to obtain an engineered bacterial strain containing the β-1,3-glycosyltransferase gene.

After activating the engineered strain containing the β-1,3-glycosyltransferase gene by plate streaking, a single colony was selected and inoculated into 5 mL of LB liquid medium containing 50 µg/mL kanamycin and incubated at 37°C for 12 h with shaking. The culture was then transferred to 50 mL of fresh LB liquid medium containing 50 µg/mL kanamycin with an inoculum volume of 2% (v/v), and then incubated at 37°C with shaking until the OD₆₀₀ reached 0.6-0.8. IPTG was added to a final concentration of 0.1 mM, and the culture was induced at 24°C for 22 h. After cultivation, the culture solution was centrifuged at 10,000 rpm for 10 min. The supernatant was discarded, and the bacterial cells were collected and stored in an ultra-low temperature refrigerator at -20°C for later use.

A 50 mM (pH 6.0) phosphate buffer solution (PBS) was prepared. The collected bacterial cells were suspended at a ratio of 1:10 (M/V, g/mL), and then high-pressure homogenization (550 Mbar for 1.5 min) was carried out. After centrifuging at 12,000 rpm for 2 min, the supernatant was collected, and the reaction enzyme solution of β-1,3-glucosyltransferase was obtained.

### Example 7: RM synthesis reaction

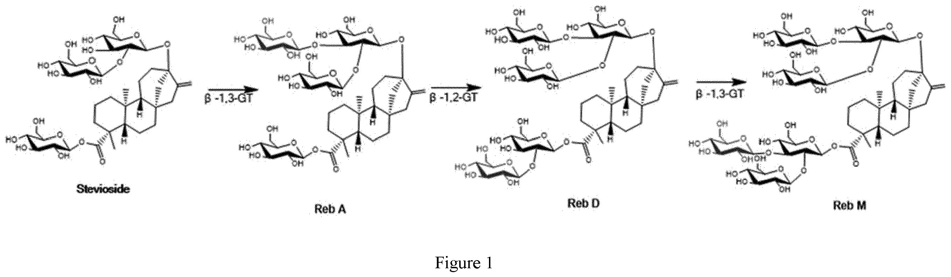

Using Reb A 60 (with a Reb A content of 60% and steviol glycosides content of approximately 30%, product specification TSG90/RA60 from ChenGuang Biotech Group) as the substrate, 120 µL of reaction enzyme solution of β-1,2-glucosyltransferase, 30 µL of reaction enzyme solution of sucrose synthase, and 150 µL of reaction enzyme solution of β-1,3-glucosyltransferase were added into 1 mL reaction system, the final concentration of RA60 is 100 g/L, the final concentration of ADP is 0.1 g/L, and the final concentration of sucrose is 200 g/L. Finally, 50 mM (pH 6.0) phosphate buffer was added to a final volume of 1 mL. The prepared reaction system was placed in a metal bath and reacted at 60°C and 600 rpm. 10 µL of the reaction solution was taken at 4 h, 7 h, and overnight, respectively, and then was added into 190 µL of hydrochloric acid with a pH of 2.0, the mixture was then vortexed, followed by 800 µL of deionized water was added and mixed well. The mixture was then centrifuged at 13,000 rpm for 10 min, and the supernatant was collected for HPLC to analyze the concentrations of Reb A, the intermediate product Reb D, and the product Reb M (RA%, RD%, and RM%). The reaction results are shown in Table 8 (wherein RA%, RD%, and RM% refer to the concentration of the substrate, intermediate product, and product in the reaction solution after the reaction, respectively). The reaction route for this example is presented in Figure 1.

**Table 8**

| Enzyme | | RD% | RM% | RA% |
|---|---|---|---|---|
| Enz.8 | Reacted for 4 h | 7.41 | 25.21 | 67.37 |
| | Reacted for 7 h | 10.01 | 41.38 | 48.61 |
| | Reacted overnight | 14.18 | 64.58 | 21.24 |
| Enz. 11 | Reacted for 4 h | 7.49 | 31.55 | 60.95 |
| | Reacted for 7 h | 9.62 | 46.86 | 43.52 |
| | Reacted overnight | 14.36 | 66.85 | 18.80 |
| Enz.14 | Reacted for 4 h | 7.20 | 31.81 | 60.99 |
| | Reacted for 7 h | 9.40 | 48.61 | 41.99 |
| | Reacted overnight | 13.46 | 70.79 | 15.76 |
| Enz.16 | Reacted for 4 h | 7.92 | 29.87 | 62.21 |
| | Reacted for 7 h | 10.19 | 44.59 | 45.23 |
| | Reacted overnight | 15.71 | 56.70 | 27.59 |

Results: from the reactions at 4 h, 7 h, and overnight, it was observed that Enz. 14 had the best reaction effect (RM%), followed by Enz. 11, and both were superior to Enz.8.

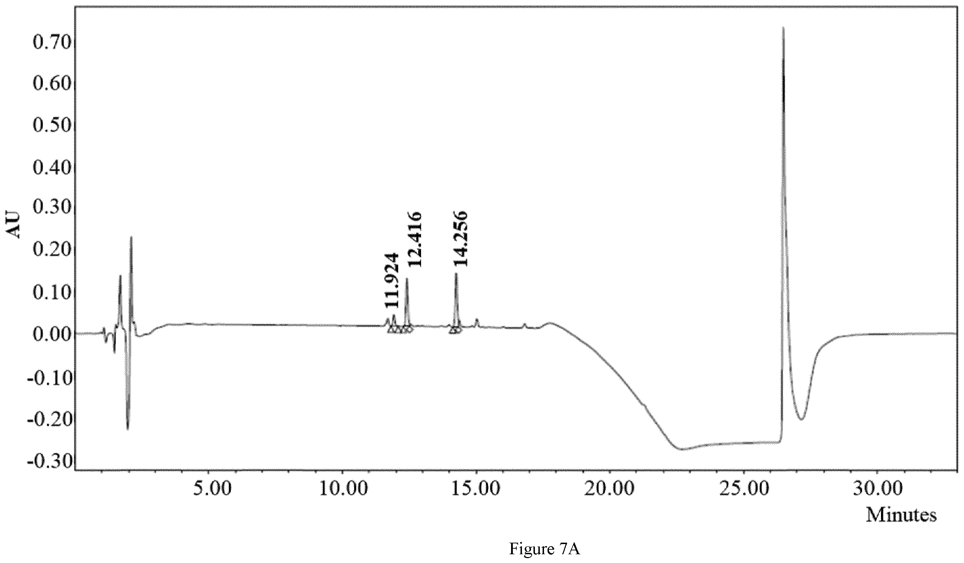

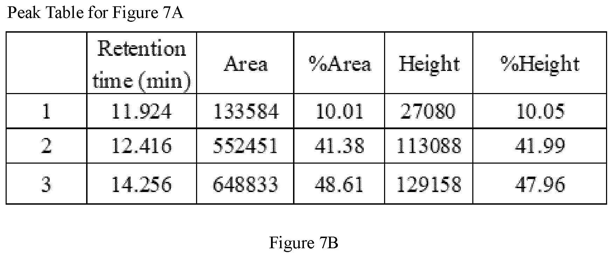

Figure 7A presents the HPLC chromatogram of the parent enzyme Enz.8 catalyzing the synthesis of RM (reaction 7 h) as listed in Table 8, while Figure 7B presents the peak information of Figure 7A.

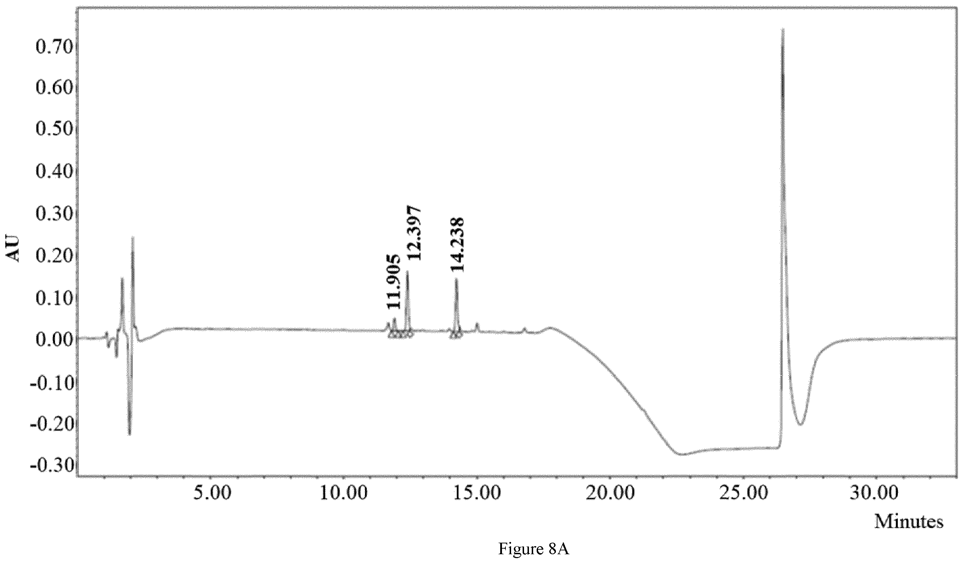

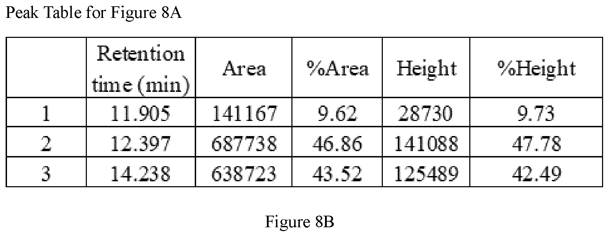

Figure 8A presents the HPLC chromatogram of Enz. 11 catalyzing the synthesis of RM (reaction 7 h) as listed in Table 8, while Figure 8B presents the peak information of Figure 8A.

## Claims

1. A sucrose synthase, wherein an amino acid sequence of the sucrose synthase comprises differences in amino acid residues selected from one or more of the following residue positions compared to SEQ ID NO: 11:
the amino acid at position 36 is V;
the amino acid at position 43 is E;
the amino acid at position 179 is A;
the amino acid at position 395 is R;
the amino acid at position 447 is P;
the amino acid at position 455 is L;
the amino acid at position 654 is R; and has sucrose synthase activity not lower than that of the amino acid sequence as shown in SEQ ID NO: 11.

2. The sucrose synthase according to claim 1, wherein the amino acid sequence of the sucrose synthase has the following differences in amino acid residues compared to SEQ ID NO: 11:
the amino acid at position 36 is V; or
the amino acid at position 43 is E; or
the amino acid at position 179 is A; or
the amino acid at position 395 is R; or
the amino acid at position 447 is P; or
the amino acid at position 455 is L; or
the amino acid at position 654 is R.

3. An isolated nucleic acid, wherein the nucleic acid encodes the sucrose synthase according to claim 1 or 2.

4. A recombinant expression vector comprising the nucleic acid according to claim 3.

5. A transformant, wherein the transformant is a host cell comprising the nucleic acid according to claim 3 or the recombinant expression vector according to claim 4; preferably, the host cell is *Escherichia coli,* for example, *E. coli* BL21 (DE3).

6. A method for preparing the sucrose synthase according to claim 1 or 2, wherein the method comprises culturing the transformant according to claim 5 under conditions suitable for expressing the sucrose synthase.

7. A composition comprising one or more of the sucrose synthases according to claim 1 or 2; preferably, the composition also comprises amino acid sequences as shown in SEQ ID NO: 9, 10, and/or SEQ ID NO: 11.

8. A method for preparing a glycosyl donor, wherein the method comprises the following steps:
reacting sucrose with nucleoside diphosphate in the presence of the sucrose synthase according to claim 1 or 2 to obtain the glycosyl donor; the nucleoside diphosphate is UDP, dTDP, TDP, CDP, IDP, GDP, or ADP;
preferably, the method for preparing the glycosyl donor satisfies one or more of the following conditions:
the sucrose synthase is present in the form of sucrose synthase bacterial cells, crude enzyme solution, purified enzyme, purified enzyme solution, or immobilized enzyme;
a mass ratio of the sucrose synthase bacterial cells to sucrose is 1:(20-200), preferably 3:200;
a mass ratio of the sucrose to the nucleoside diphosphate is 100:0.5 to 300:0.1; preferably 200:0.1;
a concentration of the sucrose is preferably 50-300 g/L, for example, 200 g/L.

9. A method for preparing rebaudioside D or rebaudioside M, wherein the method comprises the following steps: in the presence of one or more of the sucrose synthase according to claim 1 or 2, SEQ ID NO: 9, 10, and SEQ ID NO: 11, reacting rebaudioside A, glycosyltransferase, sucrose, and nucleoside diphosphate to obtain rebaudioside D;
preferably, the method for preparing rebaudioside D satisfies one or more of the following conditions:
the sucrose synthase is present in the form of sucrose synthase bacterial cells, crude enzyme solution, purified enzyme, purified enzyme solution, or immobilized enzyme;
the glycosyltransferase is present in the form of a glycosyltransferase bacterial cells, crude enzyme solution, purified enzyme, purified enzyme solution, or immobilized enzyme;
a concentration of the rebaudioside A is 1-150 g/L, preferably 50 g/L or 60 g/L;
a concentration of the sucrose is preferably 50-300 g/L, for example, 200 g/L;
preferably, a mass ratio of the sucrose synthase bacterial cells to sucrose is 1:(20-200), preferably 3:200;
more preferably, a mass ratio of the glycosyltransferase bacterial cells to the rebaudioside A is (0.1-2): 1, for example, 0.24:1 or 0.3:1;
a reaction solvent for the reaction is water, with a pH of 5 to 8, preferably at pH 6; the pH is controlled by a buffer solution, preferably a phosphate buffer solution; a rotation speed during the reaction is 500-1000 rpm, preferably 600 rpm;
a temperature of the reaction system during the reaction is 20-90°C, preferably 60°C.

10. A use of one or more of the sucrose synthases according to claim 1 or 2, SEQ ID NO: 9, 10, and/or SEQ ID NO: 11, in the preparation of steviol glycosides; the steviol glycoside is preferably rebaudioside A, D, or M.
